Europäisches Patentamt

European Patent Office

Office européen des brevets

(11) Veröffentlichungsnummer : **0 120 187**
**B1**

(12) **EUROPÄISCHE PATENTSCHRIFT**

(45) Veröffentlichungstag der Patentschrift :
30.07.86

(51) Int. Cl.⁴ : **C 12 M   3/00**, G 01 N 33/48,
G 01 N   1/10, G 01 N   1/28,
**C 12 Q   1/24**

(21) Anmeldenummer : 84100316.3

(22) Anmeldetag : 13.01.84

(54) Vorrichtung zur Aufnahme, zum Transport und zur Gewinnung von Zellmaterial sowie zur Übertragung des gewonnenen Zellmaterials auf Objektträger.

(30) Priorität : 21.03.83 DE 3310205

(43) Veröffentlichungstag der Anmeldung :
03.10.84 Patentblatt 84/40

(45) Bekanntmachung des Hinweises auf die Patenterteilung : 30.07.86 Patentblatt 86/31

(84) Benannte Vertragsstaaten :
AT CH FR GB IT LI SE

(56) Entgegenhaltungen :
DE-A- 2 244 686
DE-A- 2 917 767
DE-A- 3 248 214

(73) Patentinhaber : RHODIA AG
Engesserstrasse 8
D-7800 Freiburg (DE)

(72) Erfinder : Schlüter, Gert
Im Höfle 22
D-7803 Gundelfingen (DE)
Erfinder : Zimmermann, Matthias
Lärchenstrasse 40
D-7803 Gundelfingen (DE)

**Beschreibung**

Die Erfindung betrifft eine Vorrichtung zur Aufnahme, zum Transport und zur Gewinnung von in Körperflüssigkeiten, zellfixierenden Flüssigkeiten und Mischungen von Körperflüssigkeiten und zellfixierenden Flüssigkeiten befindlichem Zellmaterial sowie zur Übertragung des gewonnenen Zellmaterials auf Objektträger.

In der deutschen Patentanmeldung P 32 48 214.0-52 hat die Anmelderin eine Vorrichtung zur Gewinnung von Zellmaterial aus Körperflüssigkeiten, bestehend aus einem Filter mit einem in einer Schicht oder in mehreren Schichten angeordneten, in zellfixierenden Flüssigkeiten nicht löslichen Filtermaterial, vorgeschlagen. Die in der deutschen Patentanmeldung P 32 48 214.0-52 beschriebene Vorrichtung ist dadurch gekennzeichnet, daß das Filtermaterial aus einem einzigen texturierten oder gekräuselten Filamentgarn besteht ; vorzugsweise bestehen die Filamente des Filamentgarns aus synthetischen Polymeren.

Beim Einsatz jener Vorrichtung sind keine aufwendigen Zentrifugationsschritte notwendig, vermeidet man grössere Zellverluste und ist eine Fixierung der gewonnenen Zellen sofort nach der Filtration möglich.

Es stellte sich jedoch dabei heraus, daß die — übliche — Rückgewinnung des Zellmaterials aus der zellfixierenden Flüssigkeit folgende Nachteile hat :

zunächst muß das in der zellfixierenden Flüssigkeit vorhandene Zellmaterial zum Sedimentieren gebracht werden, was viel Zeit (üblicherweise mindestens 12 Stunden) in Anspruch nimmt,

nach dem Abschluß des Sedimentationsvorgangs muß die überstehende zellfixierende Flüssigkeit abdekantiert oder abgesaugt werden und

schließlich muß das Zellsediment noch mittels z. B. einer Pipette aufgenommen werden und kann dann erst auf Objektträger übertragen werden.

Der Erfindung liegt die Aufgabe zugrunde, eine Vorrichtung zur Gewinnung von in zellfixierenden Flüssigkeiten befindlichem Zellmaterial zur Verfügung zu stellen, mit welcher Vorrichtung die beschriebenen Nachteile vermieden werden.

Dabei soll diese Vorrichtung auch zur Aufnahme und zum Transport von in Körperflüssigkeiten, zellfixierenden Flüssigkeiten und Mischungen von Körperflüssigkeiten und zellfixierenden Flüssigkeiten befindlichem Zellmaterial sowie zur Gewinnung von in Körperflüssigkeiten und Mischungen von Körperflüssigkeiten und zellfixierenden Flüssigkeiten befindlichem Zellmaterial, ferner zur Übertragung des gewonnenen Zellmaterials auf Objektträger dienen können.

Diese Aufgabe wird bei einer Vorrichtung der eingangs genannten Art durch die im Kennzeichen des Anspruchs 1 angegebenen Merkmale gelöst.

Vorteilhafte Ausführungsformen der erfindungsgemäßen Vorrichtung sind in den Ansprüchen 2 und 3 angegeben.

Der Vorteil der mit Anspruch 2 beanspruchten Maßnahme liegt darin, daß sich mit ihr eine höhere Kapillarität, also eine bessere Dochtwirkung des Faserelements 5 erzielen lässt.

Der Vorteil der mit Anspruch 3 beanspruchten Maßnahme liegt darin, daß man mit ihr die Zeit, die für die Gewinnung des Zellmaterials aus der Körperflüssigkeit, der zellfixierenden Flüssigkeit oder der Mischung von Körperflüssigkeit und zellfixierenden Flüssigkeit benötigt wird, nochmals wesentlich abkürzen kann.

Verschließt man nämlich den Einlaß 2 des Gefäßes 1 mit dem Stopfen bzw. der Kappe 4 und drückt man dann das Gefäß 1 quer zu seiner Längsachse zusammen, so fließt die Flüssigkeit, in der sich das Zellmaterial befindet, beschleunigt durch das Faserelement 5 ab.

Die Hülse 8 kann als Behälter für das in der deutschen Patentanmeldung P 32 48 214.0-52 beschriebene texturierte oder gekräuselte Filamentgarn dienen und so mit ihm das dort genannte Filter bilden.

Dadurch wird es möglich, wenn man die Hülse 6 auf der dem Faserelement 5 gegenüberliegenden Seite mit dem Stopfen bzw. der Kappe 7 verschlierßt und das Gefäß 1 mit einer zellfixierenden Flüssigkeit füllt, das Filamentgarn gemäß der deutschen Patentanmeldung P 32 48 214.0-52 durch Schütteln in der zellfixierenden Flüssigkeit aufbauschen zu lassen, wodurch das dem Filamentgarn anhaftende Zellmaterial in die zellfixierende Flüssigkeit übergeht.

Durch die Hülle 9 wird verhindert, daß die Körperflüssigkeit, die zellfixierende Flüssigkeit oder die Mischung von Körperflüssigkeit und zellfixierender Flüssigkeit seitlich, also quer zur Längsachse des Gefäßes 1 und daher auch quer zur Längsachse der Fasern des Faserelements 5 aus dem Faserelement 5 austreten kann.

Für die Zwecke der Erfindung eignen sich als Fasern für das Faserelement beispielsweise solche aus

Polyolefin, wie Polypropylen,

Celluloseestern, wie Cellulose-2,5-Acetat,

Polyester, wie Polyäthylenterephthalat und/oder

Polyamid, wie Polyamid-6,6.

Wenn gekräuselte oder texturierte Fasern für das Faserelement eingesetzt werden, kommen solche in Betracht, die beispielsweise durch Stauchkammerkräuseln hergestellt worden sind.

Körperflüssigkeiten, in denen Zellmaterial vorhanden sein kann, sind z. B. Urin, Liquor, Pleuraergüsse oder Ascites.

Als zellfixierende Flüssigkeiten können z. B. Methanol, Aethanol oder Isopropanol eingesetzt werden.

Ein Ausführungsbeispiel der Erfindung wird nachstehend anhand der Zeichnungen näher erläutert. Es zeigt

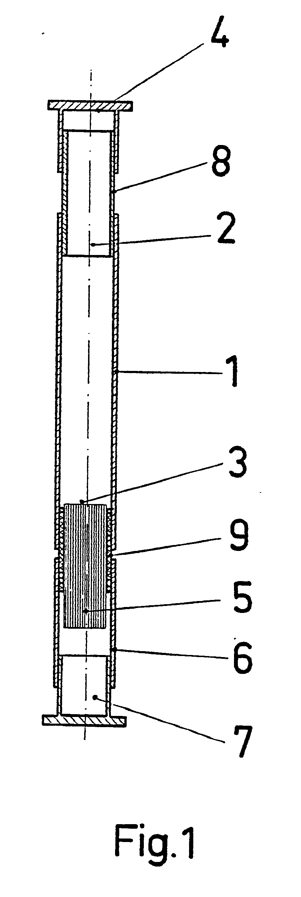

Figur 1 eine schematische Darstellung einer erfindungsgemäßen Vorrichtung im Schnitt durch die Längsachse,

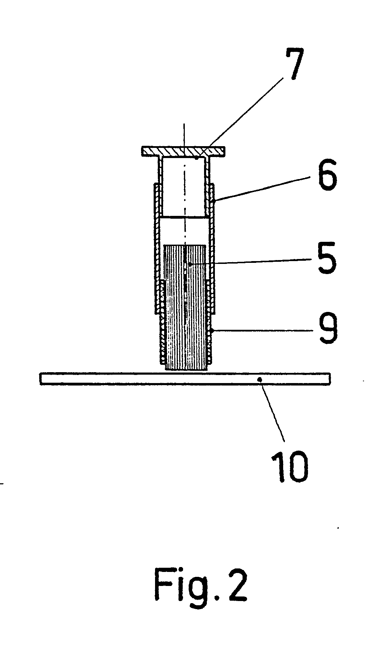

Figur 2 eine schematische Darstellung des Faserelements 5 in Zellübertragungsposition mit der mit ihm fest verbundenen Hülse 6 und dem auf der dem Faserelement 5 gegenüberliegenden Seite in der Hülse 6 eingesetzten Stopfen 7 im Schnitt durch die Längsachse.

Durch ein aus der zylindrischen Hülse 8 und einem darin befindlichen Filamentgarn bestehendes Filter gemäß der deutschen Patentanmeldung P 32 48 214.0-52 wurde Zellen enthaltender Urin filtriert. Nach Abschluß des Filtriervorgangs wurde das Filter in das Isopropanol enthaltende zylindrische, senkrecht mit dem Einlaß 2 nach oben gehaltene Gefäß 1 wandbündig eingesteckt. Die zylindrische Hülse 6 war dabei mit dem Stopfen 7 flüssigkeits- und gasdicht verschlossen. Danach wurde die Hülse 8 mit der Kappe 4 ebenfalls flüssigkeits- und gasdicht verschlossen. Sodann wurde die nunmehr geschlossene erfindungsgemäße Vorrichtung geschüttelt, wodurch das Filamentgarn sich aufbauschte, das Zellmaterial in das Isopropanol überging und gleichzeitig fixiert wurde.

Danach wurde die verschlossene erfindungsgemäße Vorrichtung mit dem darin befindlichen Zellmaterial und Isopropanol einem Zytolabor übersandt. Dort wurde die Hülse 8 mit dem Filamentgarn und die Kappe 4 von Gefäß 1 getrennt, wobei das Gefäß 1 senkrecht mit dem Einlaß 2 nach oben gehalten wurde. Danach wurde der Stopfen 7 aus der Hülse 6 herausgenommen, wodurch das Gefäß 1 mit Faserelement 5 vollständig belüftet wurde und das Isopropanol nunmehr durch die Kapillarwirkung des Faserelements 5 nach unten abfließen konnte. Die Zellen setzten sich dabei auf der dem Einlaß 2 des Gefäßes 1 zugekehrten Seite des Faserelements 5 ab. Dieser Vorgang des Abfließens und Absetzens beanspruchte eine Zeitspanne von 30 Minuten.

Sodann wurde das Faserelement 5 mit der mit ihm über die Hülle 9 verbundenen Hülse 6 aus dem Gefäß 1 herausgezogen, wonach die zelltragende Seite des Faserelements 5 mit einem Objektträger 10 in Kontakt gebracht wurde. Das gesamte auf dem Faserelement 5 abgeschiedene Zellmaterial wurde durch mehrfachen Kontakt mit dem Objektträger 10, wie bei einem Stempelvorgang, auf die Oberfläche dieses Objektträgers 10 übertragen.

Als Faserelement 5 wurde ein solches verwendet, das aus stauchkammergekräuselten Cellulose-2,5-Acetatfasern mit einem Einzelfasertiter von 2,9 dtex und einem Gesamtfasertiter von 147000 dtex bestand. Die Fasern hatten 22 Kräuselbögen pro cm, einen y-förmigen Querschnitt und in gekräuseltem Zustand alle eine Länge von 20 mm. Die Fasern waren so angeordnet, daß die beiden Enden des Faserelements 5 eine plane Oberfläche besaßen. Das Faserelement 5 war zylindrisch ausgebildet und war mit einer zylindrischen Hülle 9 aus Polyäthylen umgeben.

Das Gefäß 1 war zylindrisch, hatte eine Länge von 80 mm, einen Innendurchmesser von 12 mm und war aus Polyäthylen gefertigt. Das Gefäß 1 war quer zu seiner Längsachse zusammendrückbar.

Die Hülse 6 war ebenfalls zylindrisch, hatte eine Länge von 35 mm, einen Innendurchmesser von 12 mm und war aus demselben Material gefertigt wie das Gefäß 1.

Die Hülle 9 des Faserelements 5 hatte einen solchen Aussendurchmesser, daß sowohl mit dem Gefäß 1 als auch mit der Hülse 6 eine flüssigkeits- und gasdichte Verbindung möglich war. Die Hülse 6 war über die Hülle 9 fest mit dem Faserelement 5 verbunden.

Die Hülse 8 war auch zylindrisch, hatte eine Länge von 35 mm, einen Innendurchmesser von 8 mm und einen solchen Aussendurchmesser, daß eine flüssigkeits- und gasdichte Verbindung mit dem Gefäß 1 und der Kappe 4 möglich war. Die Hülse 8 bestand aus Polypropylen.

Die Kappe 4 bestand aus Polypropylen. Mit ihr konnte die Hülse 8 flüssigkeits- und gasdicht verschlossen werden.

Der Stopfen 7 bestand ebenfalls aus Polypropylen. Mit ihm konnte die Hülse 6 flüssigkeits- und gasdicht verschlossen werden.

Das gewonnene Zellmaterial bestand aus Zellen mit einem Durchmesser von $> 7 \, \mu m$.

Die Erfindung weist folgende Vorteile auf :

Beim Einsatz der erfindungsgemäßen Vorrichtung benötigt man zur Gewinnung von Zellmaterial aus zellfixierenden Flüssigkeiten nur einen Bruchteil der Zeit, die mit den heute üblichen Methoden und Vorrichtungen für diesen Zweck notwendig ist.

Ein Abdekantieren oder Absaugen der zellfixierenden Flüssigkeit ist nicht notwendig, da sie durch das Faserelement abfließt.

Die Übertragung des gewonnenen Zellmaterials auf Objektträger kann unmittelbar erfolgen, und zwar durch « Abstempeln », i. e. durch mehrfachen Kontakt der zelltragenden Seite des Faserelements mit der Oberfläche dieser Objektträger. Es entfällt daher die Aufnahme der Zellen mittels einer Pipette o. ä.

Außerdem dient die erfindungsgemäße Vorrichtung nicht nur der Gewinnung von Zellmaterial aus zellfixierenden Flüssigkeiten und der Übertragung der Zellen auf Objektträger, denn sie kann auch zur Aufnahme und zum Transport (beispielsweise von der Arztpraxis zum Zytolabor) von in Körperflüssigkeiten, zellfixierenden Flüssigkeiten und Mischungen von Körperflüssigkeiten und zellfixierenden Flüssigkeiten befindlichen Zellen eingesetzt werden.

Ferner kann mit der erfindungsgemäßen Vorrichtung auch Zellmaterial direkt aus Körperflüssigkeiten und aus Mischungen von Körperflüssigkeiten und zellfixierenden Flüssigkeiten gewonnen werden.

**Patentansprüche**

1. Vorrichtung zur Aufnahme, zum Transport und zur Gewinnung von in Körperflüssigkeiten, zellfixierenden Flüssigkeiten und Mischungen von Körperflüssigkeiten und zellfixierenden Flüssigkeiten befindlichem Zellmaterial sowie zur Übertragung des gewonnenen Zellmaterials auf Objektträger, dadurch gekennzeichnet, daß diese aus einem Gefäß (1) mit einem Einlaß (2) und einem Auslaß (3) besteht, wobei

a) der Einlaß (2) über eine ab- oder herausnehmbare Hülse (8) mit einem herausnehmbaren Stopfen bzw. einer abnehmbaren Kappe (4) flüssigkeits- und gasdicht verschließbar ist,

b) im Auslaß (3) wandbündig ein herausnehmbares Faserelement (5) angeordnet ist, bestehend aus in Körperflüssigkeiten und zellfixierenden Flüssigkeiten nicht löslichen Fasern, die längs in Richtung der Längsachse des Gefäßes (1) unter Bildung von durchgehenden Kapillaren so angeordnet sind, daß der Durchmesser der Kapillaren kleiner ist als der Durchmesser der Zellen, wobei das Faserelement (5) zu einem Teil seiner Länge im Auslaß (3) angeordnet ist,

c) das Faserelement (5) zum anderen Teil seiner Länge wandbündig in eine Hülse (6) hineinragt, die auf der Seite, auf der das Faserelement (5) in sie hineinragt, die gleiche Querschnittsfläche und den gleichen Querschnitt wie die des Auslasses (3) aufweist, wobei das Faserelement (5) mit der Hülse (6) flüssigkeits- und gasdicht fest verbunden ist,

d) die Hülse (6) auf der dem Faserelement (5) gegenüberliegenden Seite mit einem herausnehmbaren Stopfen bzw. einer abnehmbaren Kappe (7) flüssigkeits- und gasdicht verschließbar ist und das Faserelement (5) im Bereich des Auslasses (3) und im Bereich, in dem es in die Hülse (6) hineinragt, von einer flüssigkeits- und gasdichten Hülle (9) umgeben ist.

2. Vorrichtung nach Anspruch 1, dadurch gekennzeichnet, daß die Fasern des Faserelements (5) gekräuselte und/oder texturierte Fasern und/oder Hohlfasern sind.

3. Vorrichtung nach Anspruch 1 oder 2, dadurch gekennzeichnet, daß das Gefäß (1) quer zu seiner Längsachse zusammendrückbar ist.

## Claims

1. A device for the reception, transport and recovery of cell material present in body fluids, cell-fixing liquids and mixtures of body fluids and cell-fixing liquids as well as for the transfer of the obtained cell material to slides, characterised in that the former consists of a vessel (1) with an inlet (2) and an outlet (3) wherein

a) the inlet (2) being adapted to be closed liquid-tightly and gas-tightly with an extractable stopper or a removable cap (4), via a removable or extractable sleeve (8),

b) an extractable fibre element (5) is arranged in the outlet (3) flush with the wall, consisting of fibres which are not soluble in body fluids and cell-fixing liquids and which are arranged lengthwise in the direction of the longitudinal axis of the vessel (1), forming through capillaries, in such a way that the diameter of the capillaries is smaller than the diameter of the cells, the fibre element (5) being arranged with part of its length in outlet (3),

c) the fibre element (5) extends with the other part of its length flush with the wall into a sleeve (6) which, on the side on which the fibre element (5) extends into it, has the same cross sectional area and the same cross section as that of the outlet (3), the fibre element (5) being firmly connected with the sleeve (6) in a liquid and gas tight manner,

d) the sleeve (6) being adapted to be closed liquid-tightly and gas-tightly on the side opposite the fibre element (5) by means of an extractable stopper or a removable cap (7), and the fibre element (5) being surrounded in the zone of the outlet (3) and in the zone wherein it extends into the sleeve (6) by a liquid and gas tight sheath (9).

2. The device according to claim 1, characterised in that the fibres of the fibre element (5) are crimped and/or textured and/or hollow fibres.

3. The device according to claim 1 or 2, characterised in that the vessel (1) can be compressed transversely of its longitudinal axis.

## Revendications

1. Dispositif pour la réception, le transport et l'obtention de matière cellulaire qui se trouve dans des liquides du corps, des fluides fixant les cellules, et les mélanges des deux, ainsi que pour le transfert de la matière cellulaire sur porte-objets, caractérisé en ce qu'il est constitué d'un récipient (1) muni d'un orifice d'entrée (2) et d'un orifice de sortie (3), dans lequel

a) l'entrée (2) est prévue de façon à être fermée étanche aux liquides et aux gaz, via un tube (8) enlevable ou amovible, par un bouchon amovible ou un capuchon enlevable (4)

b) dans la sortie (3) un élément fibreux (5) amovible est fixé aux parois de cette dernière, cet élément étant constitué de fibres non solubles dans les liquides du corps et les fluides fixant les cellules, ces fibres étant placées avec leurs axes longitudinaux dans le sens de la longueur du récipient (1) de façon à former des capillaires continus conçus de telle façon que le diamètre des capillaires est inférieur au diamètre des cellules, et dans lequel l'élément fibreux (5) est fixé sur une partie de sa longueur dans l'orifice de sortie (3),

c) l'élément fibreux (5) sur l'autre partie de sa longueur est fixé aux parois d'un tube (6) à l'intérieur de celui-ci, le tube (6) présentant sur le côté où l'élément fibreux (5) est fixé la même section plane et la même section que présente la sortie (3), et l'élément fibreux (5) est fixé étanche aux liquides et aux gaz avec le tube (6),

d) le tube (6) sur le côté opposé à l'élément fibreux (5) est prévu de façon à être fermé étanche aux liquides et aux gaz, par un bouchon amovible ou un capuchon enlevable (7), et l'élément fibreux (5) du côté de sa longueur insérée pour partie dans la sortie (3) et du côté de sa longueur insérée pour partie dans le tube (6) est entouré d'un tube (9) emmanché étanche aux liquides et aux gaz.

2. Dispositif selon la revendication 1, caractérisé en ce que les fibres de l'élément fibreux (5) sont des fibres frisées et/ou texturées et/ou creuses.

3. Dispositif selon la revendication 1 ou 2, caractérisé en ce que le récipient (1) est compressible transversalement à son axe longitudinal.

Fig.1

**Fig. 2**